# EUROPEAN PATENT APPLICATION

(11) **EP 2 886 185 A1**
(43) Date of publication of application: **24.06.2015**
(21) Application number: 13198805.7
(22) Date of filing: 20.12.2013
(51) Int. Cl.: B01D 67/00, C25D 1/08, B05B 1/14, B05B 17/00, A61M 15/00, B01D 71/02

(54) **Perforated membrane and process for its preparation**

(71) Applicant: Activaero GmbH, 35285 Gemünden (DE); Temicon GmbH, 44263 Dortmund (DE)
(72) Inventor: Müllinger, Bernhard, 81373 Munich (DE); Huber, Martin, 82256 Fürstenfeldbruck (DE); Kolb, Tobias, 82061 Neuried (DE); Hoffmann, Tobias, 82005 Gilching (DE); Hartmann, Monika, 86916 Kaufering (DE); Humbach, Oliver, 44388 Dortmund (DE); Kaiser, Stefan, 44809 Bochum (DE); Gay, Nicolas, 06800 Cagnes sur mer (FR); Sauzade, Jean-Denis, 06130 Grasse (FR); Fleger, Markus, 59423 Unna (DE)
(74) Representative: Summersell, Richard John

(57) **Abstract**

The invention generally relates to the field of dispensing liquids, and in particular to aerosolizing of fine liquid droplets, for example for delivery of medicinal products to the deeper, peripheral lung. The invention provides perforated membranes with specifically shaped holes, each hole exhibiting two adjacent and preferably coaxially arranged cylindrical longitudinal sections, whose second length is larger than the first length and whose second diameter is larger than the first diameter. Such perforated membranes provide an improved hole diameter precision (± 0.5 µm), lower achievable pitch values and/or higher achievable hole densities in order to allow for the generation of very fine aerosols with droplet sizes preferably below 3 µm at high throughput rates larger than 0.5 ml/min. The invention further provides a process for the preparation of such perforated membranes and their application in inhalation devices, such as vibrating mesh nebulisers, and for inhalation therapies.

## Description

### BACKGROUND

This invention generally relates to the field of dispensing liquids, and in particular to the aerosolising of fine liquid droplets. More specifically, the invention relates to the preparation and application of perforated membranes to produce said fine liquid droplets. Distribution of oily, aqueous or alcoholic liquids, or liquid formulations (e.g. solutions, emulsions or suspensions), in the form of aerosols by means of aerosol generators is realized in a large number of applications. An aerosol is a dispersion of small solid particles or liquid droplets in a continuous gas phase, such as air. The main applications of aerosolizing liquids are delivery of bioactive agents (e.g. in medical inhalation therapies) or insecticide delivery, diffusion of cosmetic products such as perfumes or odour generation, disinfection purposes, humidification of air or substrates like paper or textiles, distribution of biological reagents, fuel combustion and inkjet printing. Although not limited to these applications, the main focus of interest in the present application lies with medical inhalation therapies and inhalation devices.

Aerosols for inhalation therapy often, but not always, comprise an active ingredient such as a drug substance useful in the prevention, management, treatment or alleviation of a disease, condition or symptom. The drug substance, often also referred to as drug, active compound, pharmaceutical, active pharmaceutical ingredient (API), or bioactive agent, may be dissolved, dispersed or suspended in a liquid - typically aqueous - carrier such as to form an aerosolisable, or nebulisable, drug formulation. Of course, the formulation may also comprise two or more drugs.

Inhalation devices such as nebulisers have been used for decades to deliver drugs for inhalation. They enable the oral inhalation of the nebulised aerosol, i.e. inhalation via the mouth, which is typically the preferred route of administration of aerosols to the lungs. In recent years, the pharmaceutical industry has become increasingly interested in drug delivery devices which transport aerosols deeper into the lungs; ideally reaching even the smallest branches of the peripheral lungs, such as bronchioles and alveoli. In particular, such devices allow administration of systemically active drugs by the respiratory route, rather than just administering locally active drugs. Improved deep lung deposition results in an optimised systemic effect and hence potential dose reductions. A further benefit of said deep lung deposition is the reduced oropharyngeal deposition, resulting in fewer drug-related side effects, such as corticosteroid-induced hoarseness or oral candidiasis.

In order to achieve the required homogeneous droplet distribution in the gas phase with a droplet size range from about 1-5 µm, the liquid formulation in the inhalation device is typically atomised by nebulisers, such as ultrasonic nebulizers, jet nebulizers or vibrating mesh nebulizers. The latter typically comprise a vibrator, such as piezoelectric element which is excited at ultrasonic frequencies in order to induce vibration; and a micro-perforated membrane, which is either permanently fixed to said vibrator by e.g. gluing, brazing, crimping, welding or detachably arranged in contact with the vibrator e.g. by a spring-coil or the like to allow vibrations of the piezoelectric element to be transmitted to the membrane.

Vibrating mesh nebulisers have outperformed more traditional devices in terms of power consumption, output rate and speed, droplet size dispersity, potential degradation of the aerosolised medicinal products and noise generation. Several manufacturers have developed inhalation devices based on vibrating micro-perforated membranes, or meshes or grids; for example, Nektar (Aeroneb^{®}), Odem (TouchSpray^{®}), Pari (e-Flow^{®}), Pfeiffer (MicroHaler^{®}), Omron (NE-U022), Sheffield Pharmaceutical (Premaire^{®}) and Alexza (Staccato^{®}). WO2008058941A1 by Télémaq describes a new type of vibrating mesh nebulizer characterized in that the micro-perforated membrane is mechanically and acoustically coupled to a piezoelectric transducer resulting in longitudinal, amplified vibratory displacements.

The first studies relating to nebulisers based on vibrating micro-perforated membranes in order to eject droplets were conducted by the research laboratory of Matsushita in the 1980s (see e.g. Ueha S. et al. "Mechanism of ultrasonic atomisation using a multi-pinhole plate", Acoust. Soc. Jpn. (E) 6.1:21 (1985); Maehara N. et al. "Influence of the vibrating system of a multipinhole-plate ultrasonic nebulizer on its performance", Review of scientific instruments, 57 (11), Nov. 1986, pp.2870 - 2876; Maehara N. et al. "A pinhole-plate ultrasonic atomizer", Ultrasonics Nov. 1984). The Matsushita patents US4533082A and US4605167A describe a piezo-electrically driven atomizer for fuel combustion or inkjet printing purposes, using a vibrating membrane perforated with 1 to 61 micro-perforations of 30 to 100 µm in diameter. The membrane is made from metal or silicone with a thickness of 30 to 100 µm and exhibits a curved part, or protuberance, (often called a dome) at its centre to facilitate divergence of the generated droplets. The membrane is glued to a vibrating piezoelectric ceramic ring which is excited at frequencies between 30 and 100 kHz to induce radial deformations in the ring. These deformations cause the membrane to deflect rearward and forward, thereby ejecting droplets through the 1 to 61 micro-perforations. This droplet ejection principle is thus also referred to as an electronic micropump.

US5152456A by Bespak adopts the principle proposed by Matsushita with the difference that the membrane, made from electroformed nickel, is not attached directly to the piezo-ceramic but to an aluminium ring disc which serves as a vibration amplifier and is in turn is fixed to the piezo-ceramic. The membrane exhibits about 1500 cylindrical, tapered micro-perforations spaced at a distance, or pitch, of 25 µm and having a diameter of 1 µm to 200 µm, for medical purposes preferably below 20 µm, e.g. 3 µm to obtain droplets of about 5 to 7 µm. However, in a typical example the proportion of said 1500 holes which actually emit droplets is only about 10 %. The membrane has a thickness of 1 to 80 µm, preferably 20 µm. Integrally formed support ribs are provided on the membrane in order to ensure sufficient stability during operation of such thin nickel sheets. The front face of is equipped with a liquid-repellent, anti-corrosive coating of e.g. PTFE particles.

US5297734A by Toda discloses an atomizer equipped with a nickel membrane of 40 to 50 µm thickness, glued to one side of a rectangular piezoelectric ceramic plate of 1 mm or 6 mm thickness. The membrane has cylindrical holes, spaced at a pitch of about 110 µm, which taper from 100 µm at their upstream end (where the liquid enters the holes) to 20 µm at their downstream end (where the liquid is ejected as droplets).

Tapering holes in a membrane used for atomizing a liquid into fine droplets are also disclosed in US6085740A and US6427682A by Aerogen. The membrane, which can be separated from the piezo-electric vibrator, exhibits holes with a diameter of 1 to 6 µm at the downstream end and a tapering slope of 10-20° relative to a central axis of the holes. The thickness ranges from about 50 µm to about 100 µm, more preferably from about 75 µm to about 100 µm to provide sufficient rigidity of the membrane. The membrane is prepared by electro-forming from palladium, palladium-alloys (palladium-cobalt or palladium-nickel), nickel or nickel-gold alloys. Optionally, the membrane may be domed; i.e. rounded outwards in the downstream direction. It may further be electroplated with gold to enhance its corrosion resistance. In another alternative, corrosion resistance of the membrane may be enhanced by a composite electroformed structure with the first electroformed layer comprising nickel and the second electroformed layer comprising gold, palladium or another corrosive-resistant metal. Other coatings improving wettability and/or preventing bacterial growth (e.g. silver, polymyxin) may also be applied.

Atomizers equipped with perforated membranes made of electroformed nickel are also described in US5261601 and US5518179 by the Technology Transfer Partnership. Optionally, a gold-coating formed by electroplating is applied to the membrane to provide water-repellent properties. The membrane is attached to a brass ring, which in turn is vibrated by the attached piezoelectric ceramic ring radially surrounding the membrane. The membrane has a thickness of 20 µm and exhibits cylindrical holes, spaced at a pitch of 50 µm, which taper from 40 µm at the upstream end to 5 µm at the downstream end. Typically, the reduction in cross-sectional area of the tapered holes from the upstream end to the downstream end is smooth and even.

In contrast, EP1022063B1 by Omron, for example, describes membranes made from metal or ceramic and exhibiting holes which - besides having conical, catenoidal or exponentially tapered shapes - can also have a so called 'stepped horn' configuration, with a large diameter opening at the internal face and a small diameter opening at the external face. However, no precise diameters, details on the membrane preparation process nor any benefits of the 'stepped horn' configuration are disclosed in EP1022063B1.

Similar 'stepped' through-holes, or so-called holes with a 'multistage-structure', are also disclosed in US7972543B2, which does not relate to membranes for vibrating mesh nebulisers but to electroformed perforated metal substrates for the use in emulsion preparation processes. These metal substrates are more stable and durable than the commonly used silicone filters. The metal substrates have a thickness of 10 µm to 6,000 µm and exhibit through-holes with a diameter of 0.5 to 500 µm. The diameter-to-thickness ratio ranges from 1:1 to 1:30. The through-holes can have circular, ellipsoidal or rectangular cross sections. One specifically depicted embodiment shows a metal substrate with 'multistage-structured' through-holes, spaced 90 µm apart, measuring 60 x 20 x 20 µm (length x width x depth, respectively) at the upstream end and 20 x 20 x 150 µm at the downstream end. This 'multistage-structure' of the through-hole is chosen for improving the microsphere production efficiency. None of the disclosed embodiments would provide membranes suitable for vibrating mesh nebulisers and/or yield droplet sizes suitable for inhalation.

It is known to the skilled person that the shape and size of the holes in the membrane employed in a nebuliser have an influence on e.g. throughput, droplet size and polydispersity of the aerosol. In particular, the hole diameter and shape at the downstream face of the membrane, where the aerosol droplets are expelled, determines the size of the droplet. With current state of the art membranes exhibiting circular holes, the diameter of the droplets is typically about 2 times the diameter of the hole. For example, in US6235177, droplets of about 2 µm to 10 µm diameter were obtained using a membrane with tapered holes of 1 µm to 10 µm diameter at the narrowest point of the taper. This is likely caused by the Plateau-Rayleigh instability which causes a fluid jet to break up into droplets having twice the diameter of the fluid jet upon exiting a nozzle.

Various processes have been described for the preparation of perforated membranes used for aerosol generators, such as drilling, laser drilling, etching, press-punching, electroforming, or electroplating. An example of an electroforming process uses the so-called overgrowth method for electrodepositing metals, such as nickel or palladium or alloys, on a substrate or mandrel. Electroforming techniques are described generally in E. Paul DeGarmo, "Materials and Processes in Manufacturing" McMillan Publishing Co., Inc., New York, 5th Edition, 1979, the complete disclosure of which is herein incorporated by reference.

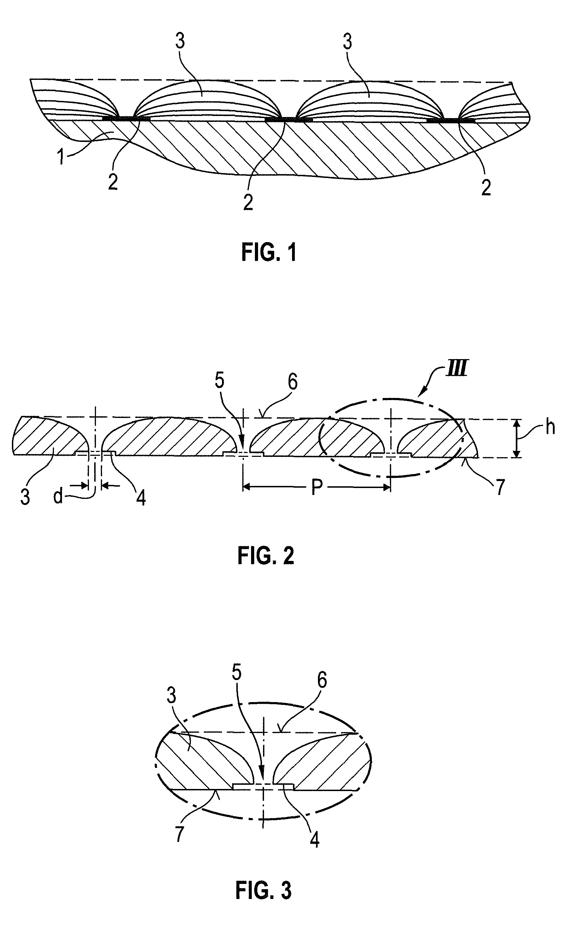

Two types of electroforming processes are known in the state of the art, referred to as 'thin resin' and 'thick resin' process. The processes are summarized e.g. in EP1199382A1. In the 'thin resin' process, a thin insulating photosensitive layer is applied onto an electrically conductive substrate, such as a metal or silicon wafer, to a thickness of typically about 1 µm to 5 µm, sometimes up to 20 µm. Typically, a so called negative photoresist is used as the photosensitive layer, due its superior adhesion properties. Using ordinary photolithographic techniques, such as UV-exposure through laser-cut, insulating exposure masks (typically glass or quartz), the negative photoresist is solidified and thus adheres to the substrate in a desired pattern and shape. In a subsequent development step, the unexposed portion of the photoresist is dissolved and removed from the electrically conductive substrate, leaving behind patterned lozenges, or islets, of solidified photoresist. As illustrated in figure 1, an electro-formable material (3), such as a metal, is then electrodeposited, or electroplated, onto the electrically conductive substrate (1), gradually filling the gaps between the patterned lozenges of photosensitive material (2), and finally overgrowing them isotropically. Figure 1 shows a cross-sectional view of the different stages of deposition of electro-formable material (3) on the electrically conductive substrate (1). The electro-formable material (3) is allowed to grow by electrodeposition until the desired shape (thickness, hole diameter) of the membrane is reached. Then the electrically conductive substrate (1) and the patterned photosensitive material (2) are removed, e.g. by peeling or chemical dissolution, to obtain a perforated membrane of electro-formable material (3) with holes (5) as illustrated in figure 2. An enlarged detail of a hole (5) as formed by the prior art 'thin-resin' process is shown in figure 3.

Optionally, a curing step can be employed after the exposure step in order permit the patterned lozenges, or islets, of solidified photoresist to flow and cure in a desired shape. For example, the islands may be heated until they are generally conical or dome shaped and have a slope relative to the surface of the substrate. This is described, for instance, in US6235177 which claims a method of manufacturing a micro-perforated membrane by means of electrodeposition of palladium nickel alloys (or other palladium alloys), the membrane being characterized by a network of at least 1000 conically tapered holes with a diameter of 1 to 10 µm, preferably 5 µm; and a tapering angle between 30° and 60°, most preferably about 45°.

Corrosion-resistant and/or water-repellent coatings may be applied using the same electrodeposition process before the electrically conductive substrate and the patterned photosensitive material are removed. This is described e.g. in WO2003028895A2 which uses materials such as nickel alloys, nickel manganese, copper, tin and the like as cheaper base materials; and the more expensive corrosion resistive materials such as palladium, platinum, rhodium, palladium cobalt, gold and the like only as a thin coating, covering the internal face and the tapered part of the holes.

Due to the underlying overgrowth mechanism, the 'thin resin' process inevitably produces trumpet-shaped, flared micro-perforations. Each hole has a curved interior shape whose radius is approximately equal to the thickness of the membrane (h) and the holes (5) taper from a larger diameter at the upstream end (6) to a smaller diameter at the downstream end (7). A drawback of 'thin resin' process type membranes lies in their overall limited throughput. The flow through the membrane correlates directly to the square of the hole diameter. Hence, in order to allow for small hole diameters (as typically required for droplet sizes suited to inhalative purposes) without reducing the flow, it would be necessary to increase the number of holes per surface area; i.e. decrease the distance between the holes, or pitch (P). However, there is a relationship between the diameter of the holes (d), the pitch (P) and the thickness (h) of the membrane, which can be seen in figures 1 and 2. The shape and dimensions of the holes (5) are the result of the thickness (h) (as determined by the duration of the electrodeposition process), the pitch (P) (as determined by the distance between the islets, or lozenges, of photosensitive material) and the specific pattern of the islets, or lozenges, of photosensitive material (e.g. hexagonal or rectangular grids). It is, for example, impossible to reduce the pitch (P) to less than twice the thickness (h). The thickness (h) on the other hand cannot be freely decreased since the membranes require a certain thickness to be stable to be handled safely during manufacture, transport and assembly of the aerosol generator and withstand the vibrational forces during their use. Thus, the flexibility with regard to diameter, pitch and thickness, and consequently performance, of membranes prepared by the 'thin resin' process is rather limited. It is further generally accepted that the 'thin resin' process allows for holes with diameter tolerances of ± 1 µm and therefore does not permit sufficient precision for holes smaller than 3 µm. This level of precision is not satisfying when e.g. monodisperse aerosols characterized by a geometric standard deviation (GSD) of less than 1.2 are required for certain therapeutic applications.

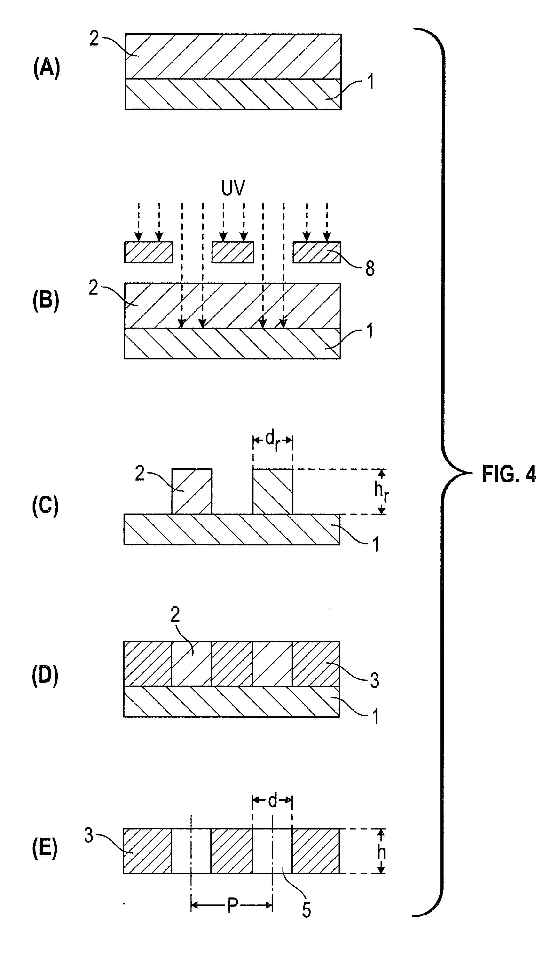

The second electroforming process known in the state of the art is the so called 'thick resin' process, as illustrated by figures 4A to 4E (modified from EP1199382A1). In this process, a photosensitive material (2), such as a negative photoresist, is deposited as a relatively thick layer onto an electrically conductive substrate (1) (figure 4A). Then, the photosensitive material (2) is exposed selectively to e.g. UV-light through an insulating exposure mask (8) so as to cause the photoresist (2) to solidify and adhere to the substrate (1) in the desired pattern and shape (figure 4B). (Typically, the electrically conductive substrate (1) is UV-opaque, hence the UV-exposure step of the applied photoresist (2) is performed from the top, or front; a process sometimes referred to as front-exposure.) Then, the photosensitive material (2) is developed, removing the unexposed fractions of the photoresist (2) and thus forming a pattern of photoresist columns, whose height (hᵣ) is determined by the thickness of the photosensitive material (2) layer applied to the electrically conductive substrate (1) (figure 4C). The membrane made of electro-formable material (3) and comprising the holes (5) is again formed by electrodeposition on the substrate (1), gradually filling the gaps between the patterned photoresist columns (2) (figure 4D). The electro-formable material (3) is allowed to grow on the electrically conductive substrate (1) until the desired thickness of the membrane (h) is reached; maximally until the height of the deposited electro-formable material (3) reaches the height (hᵣ) of the columns of photoresist (2). After the electro-deposition step, the electrically conductive substrate (1) and the photoresist (2) are removed to obtain a membrane of electro-formable material (3) of a desired thickness (h), with through-holes (5) spaced at a desired pitch (P) and having a desired diameter (d) (figure 4E).

From figures 4A to 4E it can be seen, that, in principle, the 'thick resin' process allows the formation of thicker membranes - stable enough for application in vibrating mesh nebulisers - without influencing the hole diameter (d) and pitch (P) at the same time. The hole diameter (d) and pitch (P) are, in theory, controllable by the shape and dimensions of the patterned photoresist (2). However, as described in EP1199382A1, the diameter (d) and length, or depth, of the through-holes (5) which could be formed with the 'thick resin' process is nonetheless limited because it was empirically found that the columns of photoresist (2) tend to tilt, topple over and/or break if the height of the column (hᵣ) exceeded its diameter (dᵣ). In consequence, most prior art 'thick resin' processes yielded membranes with through-holes whose depth was smaller than their diameter. One potential explanation is that the UV-intensity gradually decreases while passing through the thick layer of the photosensitive material, so that the resulting photoresist columns get decreasingly solidified towards their bottom end, or base (i.e, towards the side of the electrically conductive substrate). This may destabilize the photoresist columns.

By a modification of the UV-exposure process, membranes with tapered through-holes having a depth (i.e. membrane thickness) larger than the diameter (d) could be obtained in EP1199382A1. This was achieved by changing the process from the typical front-exposure (where the thick photoresist layer is applied directly to an un-patterned and UV opaque electrically conductive substrate and then exposed to UV from the top, or front) to a back-exposure process. In the back-exposure process of EP1199382A1, a transparent substrate such as a thin borosilicate glass is first sputter-coated with a thin electrically conductive and UV-opaque layer of e.g. chromium and/or gold. Then a pattern is formed in said UV-opaque layer by etching, thus forming the UV-exposure mask. In one example provided in EP1199382A1, circular holes 20 µm in diameter are spaced at a pitch of 40 µm. Only then the thick layer of photosensitive material is applied onto the transparent substrate (approximately 60 to 110 µm thick). This means that instead of placing a patterned UV-exposure mask over the photosensitive material, the mask is sandwiched between the transparent substrate and the photosensitive material. Then, UV-exposure is performed from the back, with the UV light passing the transparent substrate and selectively passing the electrically conductive, UV-opaque exposure mask. Again, the negative photoresist is solidified in the areas of UV-exposure, thereby forming photoresist columns with a diameter of e.g. 20 µm and approximately 60 to 110 µm high, while the unexposed fractions remain soluble and will be removed in the development step. Using this photoresist, membranes with holes of e.g. 20 µm in diameter and about 60 µm to 110 µm in thickness can be obtained when electrodepositing the electro-formable material in a typical manner. According to EP1199382A1, thicknesses of up to 15 times the diameter of the holes can theoretically be achieved, requiring however that the process must be repeated numerous times, which is hardly feasible at the required precision and which increasingly complicates the removal of the photoresist upon each repetition. Moreover, such process would not be cost-efficient.

According to EP1199382A1, the reason why the photoresist columns do not tilt or topple over despite their larger height in comparison to their diameter can presumably be found in the favourable UV-scattering during back-exposure: UV radiation is diffracted and bent inwards at the edges of the exposure mask, and in addition the UV-exposure gradually decreases when passing through the thick photosensitive material layer. In consequence, the formed photoresist columns are not cylindrical but have an either torpedo-like or frustoconical shape, tapering from a wider diameter (given as diameter 1 in table 1 at their bottom end (on the substrate) to a smaller diameter (diameter 2) at their top end, sometimes even yielding photoresist columns with pointy tips. Furthermore, since the UV-intensity is the highest near the transparent substrate during back exposure, the photosensitive material solidifies best at the bottom end, providing a stable base to the photoresist column. This prevents or at least limits their tendency to tilt, topple over and/or break to some degree; and at the same time inevitably yields tapered holes in membranes during the electrodeposition step.

The main disadvantage of this back exposure process is that the precise shape of the photoresist columns (and in consequence the tapering angle of the holes and their diameters) results from the material-inherent UV-scattering in the photoresist layer and thus is a complicated function of e.g. photoresist material, thickness of the applied photoresist layer, UV-density, exposure time. The shape of the holes in the electroformed membrane can only be controlled to some degree by choosing a specific membrane thickness in combination with a defined height of the photoresist columns. For example, when depositing the electro-formable material up to (almost) the complete height of the photoresist columns, the tapering angle increases with membrane thickness photoresist column height as shown in table 1.

**Table 1**

| Membrane thickness | Diameter 1 | Diameter 2 | Tapering angle |
|---|---|---|---|
| 25 µm | 8 µm | 7.5 µm | 0.57° |
| 40 µm | 10 µm | 9 µm | 0.72° |
| 50 µm | 20 µm | 18 µm | 1.15° |
| 100 µm | 20 µm | 2 µm | 5.14° |

If, on the other hand, a comparably thin membrane is deposited on high photoresist columns, the tapered shape of the photoresist columns does not have as much impact anymore. For example, a membrane with a thickness of 30 µm prepared on a photoresist with columns of 110 µm height exhibited un-tapered holes (diameter 20 µm on both sides of the membrane). In consequence, it requires a lot of testing and optimization efforts in order to use the back-exposure 'thick resin' process of EP1199382A1 to prepare membranes having a specifically desired thickness and at the same time controllable hole diameters at the membrane's two sides, e.g. the upstream and downstream sides of the membrane when used in a vibrating mesh nebuliser.

It is thus an object of the current invention to provide an improved perforated membrane which overcomes any of the drawbacks and limitations of the prior art. In particular, it is an object to provide an improved perforated membrane which may be used as a vibratory membrane in an aerosol generator or in an inhalation device useful for inhalation therapy. Moreover, it is an object to provide a membrane which allows for lower pitch values than achievable according to the prior art, and/or for higher hole densities than achievable in the past, such as to allow for the generation of very fine aerosols at high throughput rates.

It is a further object to provide a membrane with an improved precision of hole diameters.

A yet further object of the invention is to provide a process for the preparation of such perforated membrane which overcomes any of the drawbacks and limitations of the conventional manufacturing methods.

In another aspect, it is an object of the invention to provide an improved aerosol generator and/or inhalation device which does not exhibit one or more of the disadvantages and limitations of the prior art devices.

Yet further objects will become clear on the basis of the following description, the drawings, the examples, and the claims.

### SUMMARY OF THE INVENTION

Objects of the invention are met by the perforated membrane according to claim 1, a preparation process for said membrane according to claim 14, an inhalation device with said perforated membrane according to claim 11 and the use of said perforated membrane and/or the inhalation device for inhalation therapy according to claim 13. Advantageous embodiments are provided in the dependent claims.

In particular, a perforated membrane is provided which comprises a region with at least 50 holes/mm², each hole having a first longitudinal section being shaped as a right circular cylinder with a first length and first diameter, and an adjacent second longitudinal section being shaped as a right circular cylinder with a second length and second diameter, wherein the second length is larger than the first length and the second diameter is larger than the first diameter. The first and second longitudinal sections are arranged in such a way that the base of the first longitudinal section facing the second longitudinal section is contained in the base of the second longitudinal section facing the first longitudinal section. The first diameter is from 1 µm to 5 µm and the first length is not more than 20 µm, and the second diameter is from 4 µm to 50 µm. Moreover, the aspect ratio of the first length divided by the first diameter is at least about 2, or at least about 2.5, respectively.

Further, the perforated membrane may be made from a metal selected from the group of nickel, palladium, cobalt or an alloy of at least two members thereof.

In one particular embodiment, the first diameter is in the range from 1.5 µm to 4 µm and the first length is in the range from 5 µm to 10 µm, and the second diameter is optionally in the range from 20 µm to 30 µm.

In one embodiment, the perforated membrane has a hole density in the range from 100 to 400 holes/mm² in the region.

In one embodiment, the perforated membrane has ratio of the pitch to the first length in the range from about 2.5 to about 25, and the pitch is equal to the thickness of the membrane or less.

In a further embodiment, the region comprises only holes of the same first diameter. Alternatively, the region comprises at least two different sets of holes having different first diameters.

In a further embodiment, the perforated membrane comprises holes exhibiting a sharp edge at their downstream end.

In a further embodiment, the perforated membrane at least partially forms a dome in a central position, such that the dome comprises the region.

In a further embodiment, the perforated membrane further comprises a water-repellent, biocompatible and/or corrosion-resistant coating.

The invention further provides an inhalation device comprising the perforated membrane described above. In particular, an inhalation device is provided wherein the perforated membrane is coupled mechanically and acoustically to a piezoelectric transducer.

The invention further provides the use of the perforated membrane or of the inhalation device for inhalation therapy.

The invention further provides a process for preparing the above mentioned perforated membrane by electro-forming comprising the steps of
(A) coating a silicon wafer with a conductive seed layer,
(B) preparing a first patterned photoresist layer by
   i) spin coating the product of step (A) with a thin layer of a first photosensitive material having a first thickness,
   ii) exposing said first photosensitive material to UV-light through a laser-cut mask to solidify the first photosensitive material in a first predetermined pattern
   iii) chemically developing the first photosensitive material to obtain a predetermined pattern of first right circular cylinders having a first diameter and a first length
(C) preparing a first layer of the perforated membrane by electro-depositing an electroformable material using the first patterned photoresist layer of step (B) until the thickness of said first layer approximately reaches the first length of the photoresist cylinders,
(D) preparing a second patterned photoresist layer by
   iv) laminating the product of step (C) with a layer of a second photosensitive material having a second thickness larger than the thickness of the layer of the first photosensitive material
   v) exposing said second photosensitive material to UV-light through a laser-cut mask to solidify the second photosensitive material in a second predetermined pattern
   vi) chemically developing the second photosensitive material to obtain a predetermined pattern of second right circular cylinders adjacent to the first right circular cylinders and having a second length and a second diameter, wherein said second length is larger than the first length and said second diameter is larger than the first diameter, and wherein the first and second right circular cylinders are arranged in such a way that the base of the first right circular cylinder facing the second right circular cylinder is contained in the base of the second right circular cylinder facing the first right circular cylinder
(E) rinsing the product of step (D) with acid
(F) preparing a second layer of the perforated membrane, adjacent to the first layer, by electro-depositing an electro-formable material using the second patterned photo resist layer of step (D) until the thickness of said second layer approximately reaches the second length of the photoresist cylinders,
(G) removing the silicon wafer along with the conductive seed layer,
(H) removing the photoresist materials in a dry chemical etching process.

In one embodiment of the process, the conductive seed layer is a composite of two or more separate layers made of materials selected from chromium, titanium, silver, copper, gold and/or aluminium; and/or the first photosensitive material is a liquid negative photosensitive material chosen from SU-8, AR-N 4400 or AZ9260; and/or the second photosensitive material is a dry negative photosensitive material chosen from Ordyl FP400 or Ordyl SY300; and/or the acid is chosen from sulphuric acid or sulphamic acid; and the dry chemical etching process is an oxygen based plasma stripping process.

Other objects, features and aspects of the present invention are disclosed in or are obvious from the following detailed description.

### DEFINITIONS

The following expressions as used herein should normally be interpreted as outlined in this section, unless the description provides a different meaning in the text or in a specific context.

Unless defined otherwise, all technical and scientific terms used herein have the same meaning as commonly understood by one of ordinary skill in the art to which this invention belongs.

All terms and expressions designating a position, orientation or direction, such as upper, lower, left, right, front, back, top, bottom, up, down, superior, inferior and the like, should be understood with reference to the orientation of the inhalation device or its components under normal operational conditions, and typically from the perspective of the user.

Generally the term "circular cylinder" refers to a geometric, three-dimensional shape defined by two parallel, circular bases, which may also be referred to as front faces, or as top and bottom surface, of approximately the same diameter d, and a rectangular curved surface area formed by all points located at a fixed distance (half the diameter d) from the centre axis of the cylinder. In case of a right circular cylinder, the centre axis is substantially perpendicular to the bases; i.e. a right cylinder is not tilted. Of course, these geometric definitions describe an ideal form. In the context of the invention, however, the skilled person will understand that the real geometries will always differ from the ideal. For example, due to the inherent limitations of some of the manufacturing processes described herein, the shapes obtained in the final product may vary slightly from the strict geometric definitions. Thus, as used herein, the term "right circular cylinder" means a geometric, three-dimensional shape defined by two parallel, substantially circular bases having substantially the same diameter d; and a rectangular curved area formed by all points located at a fixed distance (substantially half of said same diameter d) from the centre axis of the cylinder, with said centre axis being substantially perpendicular to the two parallel, substantially circular front faces. In this regard, "substantially circular" refers to circular shapes with an aspect ratio of largest diameter and smallest diameter orthogonal to it between 1 (perfect circle) and about 1.3 (slightly elliptical); "substantially same diameter" would include circular shapes whose diameters differ somewhat, such as by 10%, or even 15% or even more, taking into account the common technical variability in the particular technical field and the scale.

As used herein, "contained in" means, with respect to the base of the first longitudinal section facing the second longitudinal section being contained in the base of the second longitudinal section facing the first longitudinal section, the two specified bases are in approximately the same plane and that the respective base of the first longitudinal section does not extend beyond the boundaries of the respective base of the second longitudinal sections, at least not to a technically significant degree.

"Upstream", with respect to the membrane or a hole in the membrane, refers to the side, face or end which, during operation in an inhalation device such as a vibrating mesh nebuliser, faces the liquid to be nebulised and/or is in direct contact with said liquid. The upstream side of the membrane may also be referred to as the internal face, or rear face or rear side or back side.

"Downstream", with respect to the membrane or a hole in the membrane, refers to the side, face or end from which aerosolised droplets are expelled or ejected. The downstream side of the membrane may also be referred to as the external face, or front face or front side.

It is to be understood that the use of expressions like upstream and downstream, or external and internal face, with regard to the application of the perforated membrane according to the invention in inhalation devices, such as vibrating mesh nebulisers, is not intended to limit the invention to inhalation devices in general or to vibrating mesh nebulisers in particular. Perforated membranes according to the invention may be used in any field of liquid dispensing, such as in apparatus for spray drying, spray congealing, or filtration. Thus, if considered more suitable, the perforated membrane may also be turned around into an orientation where the liquid to be dispersed enters the narrower, first part of the holes first.

The term hole, or through-holes or aperture or perforation or micro-perforation, refers to the openings in the membrane through which liquid is ejected in the form of droplets.

The term "pitch", or inter-hole distance, describes the distance between the holes as measured from the centre of one hole to the centre of the neighbouring hole.

As used herein, the "dome", or protuberance or bulge, is a curved, or bent, part of the membrane which curves the membrane in a convex manner in the downstream direction. The dome is typically located in a central position of the membrane and comprises a region with holes.

The term "photoresist" can have two similar meanings, depending on the context. On the one hand, it is used synonymously to "photosensitive material", or "photoresist material", as it is applied e.g. in photolithographic processes to form patterned coatings on surfaces. Photosensitive materials, or photoresist materials, typically comprise a photosensitive component in a base of polymer(s) (e.g. polymethylmethacrylate, Novolak, polymethylglutarimid) or epoxy resin (e.g. SU-8) in solvents like cyclopentanone or gamma-butyrolactone. Negative photoresists become insoluble to the photoresist developer e.g. by cross-linking; i.e. they harden or solidify when exposed to (UV)-light, such that in a subsequent developing step only unexposed portions of the photoresist can be dissolved and removed. Often, negative photoresists are preferred over positive ones (where the exposed parts become soluble to the photoresist developer), due to their superior adhesion properties to electrically conductive substrates such as silicon. Thus, unless specified otherwise, the term "photoresist" as used herein refers to the negative type of photosensitive materials. On the other hand, the term "photoresist" can also refer to the hardened, patterned fraction of the photosensitive material that is obtained after the exposure and development steps and which serves as a mould in the electrodeposition step.

The expression "electro-formable material" generally refers to any kind of material which can be deposited by electroplating, specifically electro-forming. This includes e.g. nickel, palladium, platinum, gold, manganese, rhodium, silver, copper, cobalt, tin, zinc, lead and their alloys. Most commonly used to prepare membranes for vibrating mesh nebulisers are nickel, palladium, cobalt, gold and their alloys.

The expressions "sharp edge" or "sharp edge definition" refer to edges that are substantially free of roundings, splays, chamfers or imperfections like protruding ridges and/or indentations and which would thus qualify for edge values of +0.05 or lower in technical drawings following DIN ISO 13715 (German Industry Norm).

The expression "mechanically and acoustically coupled" means that the perforated membrane according to the present invention is mechanically attached to a piezoelectric transducer in such a way that it is capable of functioning as an 'active' perforated membrane (or vibratory membrane, or vibrating membrane, or vibrating mesh); i.e. the perforated membrane is acoustically coupled and thus vibrates in response to the vibrations transferred to it from the piezoelectric transducer. Not all methods to mechanically couple, or attach, or fix, a perforated membrane to a piezoelectric transducer are equally suited to also allow for acoustic coupling; i.e. not all mechanical coupling options sufficiently transfer the vibrations of the piezo-element to the perforated membrane in order to induce active vibration. Out of the various options, gluing, brazing and welding (e.g. laser welding) are preferred in order to ensure proper acoustic coupling. Other options such as crimping or link members (e.g. threaded means for wedging the membrane on the transducer; alone or in combination with washers and/or spring coils) are feasible, too, but should be evaluated more carefully with regard to their acoustic coupling performance.

An "aerosol generator" is a device or device component capable of generating an aerosol.

A "nebulising means" is an aerosol generator which generates an aerosol from a liquid. The aerosol comprises a liquid phase consisting of small, typically inhalable droplets dispersed in a gas phase such as air.

As in the current technical literature, a "nebulizer" either refers to a nebulizing means or to an inhalation device comprising a nebulization means, depending on the context.

"Comprise" or "comprising", with reference to any feature, means that the respective feature must be present, but without excluding the presence of other features.

"A" or "an" does not exclude a plurality unless the context clearly dictates otherwise.

"Essentially", "about", "approximately", "substantially" and the like in connection with an attribute or value include the exact attribute or the precise value, as well as any attribute or value typically considered to fall within a normal range or variability accepted in the technical field concerned; e.g. ± 15 %.

Any reference signs in the claims should not be construed as a limitation to the embodiments represented in any of the drawings.

A single unit may fulfil the functions of several features recited in the claims.

### BRIEF DESCRIPTION OF THE DRAWINGS

**Figure** 1 shows a cross-sectional view of the manufacturing process of a perforated membrane according to prior art using the 'thin resin' electro-deposition process.
**Figure 2** shows a cross-sectional view of the perforated membrane according to prior art, as obtained by the 'thin resin' electro-deposition process depicted in figure 1.
**Figure 3** shows an enlarged detail of the area III in figure 2.
**Figures 4A to** E show cross-sectional views of the different steps of the manufacturing process of a perforated membrane according to prior art using the 'thick resin' electro-deposition process (figures modified from EP1199382A1).
**Figure 5** shows a cross-sectional view of a perforated membrane according to an embodiment of the invention.
**Figures 6A and B** show top views of two perforated membranes, both comprising a region with holes according to embodiments of the invention.
**Figures 7A and B** show enlarged cross-sectional views of a hole in a perforated membrane according to the invention, the hole exhibiting either a sharp edge definition (A) or a chamfered or rounded edge (B).
**Figure 8** shows a side view of a perforated membrane according to the invention comprising a dome.
**Figure 9** shows a cross-sectional view of an exemplary embodiment of an inhalation device equipped with a perforated membrane according to the invention.
**Figure 10** shows a cross-sectional view of an exemplary embodiment of a piezo-electrically driven nebulising means equipped with a perforated membrane according to the invention.
**Figures 11A to** I show the different steps of the manufacturing process of a perforated membrane according to the invention in a cross-sectional presentation.

**LIST OF NUMERICAL REFERENCES USED IN THE FIGURES**

| | | | |
|---|---|---|---|
| 1 | Electrically conductive substrate | 18 | Dome |
| 2 | Photosensitive material | 19 | Inhalation device |
| 3 | Electro-formable material | 20 | Power cable |
| 4 | Recess | 21 | Liquid reservoir |
| 5 | Hole | 22 | Nebulising means |
| 6 | Internal face | 23 | Stress concentration zone |
| 7 | External face | 24 | Vibration displacement amplification zone |
| 8 | Exposure mask | 25 | Annular piezo-element |
| 9 | Perforated membrane | 26 | Silicon wafer |
| 10 | Region | 27 | Conductive seed layer |
| 11 | First longitudinal section | 28 | First photosensitive material |
| 12 | Second longitudinal section | 29 | First patterned photoresist layer |
| 13 | Annular outer part | 30 | First layer of the perforated membrane |
| 14 | Circular centre part | 31 | Second photosensitive material |
| 15 | Sharp edge | 32 | Second patterned photoresist layer |
| 16 | Droplet | 33 | Second layer of the perforated membrane |
| 17 | Edge with roundings, splays, chamfers or imperfections | | |

### DETAILED DESCRIPTION OF THE INVENTION

The invention provides, inter alia, a perforated membrane (9) which comprises a region (10) with at least 50 holes/mm², each hole (5) having a first longitudinal section (11) being shaped as a right circular cylinder with a first length (h₁) and first diameter (d₁), and an adjacent second longitudinal section (12) being shaped as a right circular cylinder with a second length (h₂) and second diameter (d₂), wherein the second length (h₂) is larger than the first length (h₁) and the second diameter (d₂) is larger than the first diameter (d₁). The first and second longitudinal sections (11, 12) are arranged in such a way that the base of the first longitudinal section (11) facing the second longitudinal section (12) is contained in the base of the second longitudinal section (12) facing the first longitudinal section (11). The first diameter (d₁) is from about 1 µm to about 5 µm and the first length (h₁) is not more than about 20 µm. The second diameter (d₂) is from about 4 µm to about 50 µm. The membrane is further characterised in that the aspect ratio of the first length (h₁) divided by the first diameter (d₁) is at least 2. Optionally, the aspect ratio of the first length (h₁) divided by the first diameter (d₁) in a perforated membrane (9) according to the invention is at least about 2.1, or 2.2, or 2.3, or 2.4, or 2.5, respectively. In another embodiment, the aspect ratio is at least about 3.

In addition to the region defined above, the membrane may exhibit one or more further regions with holes.

A major advantage of the invention is that perforated membranes (9) can be prepared with a thickness (h) sufficient to withstand the conditions during manufacture, assembly and use, e.g. vibrations, while at the same time allowing very small diameters (d₁) of the holes (5) at the external face (7). Moreover, the invention enables membranes with holes spaced at a low pitch (P), which allows for a high hole density and thereby a high throughput, or rate of aerosol generation.

Moreover, it was found by the inventors that the membrane of the invention exhibits only very little undesirable dripping of liquid through the non-vibrating membrane, which is a common problem associated with high pitch membranes according to prior art.

Preferably, the first and second longitudinal sections (11,12) forming the hole(s) (5) are arranged in such a way that their respective centre axes are substantially parallel to each other. In particular, the first and second longitudinal sections (11,12) forming the hole(s) (5) in a perforated membrane (9) according to the invention may be arranged coaxially; i.e. having the same centre axis. It should be noted that while such a coaxial, or substantially coaxial, arrangement of the first and second longitudinal sections (11,12) is an important embodiment of the invention, the inherent limitations of some of the manufacturing processes described herein may also lead to holes (5) where the centre axes of the first and the second longitudinal sections are displaced, or off-centred, without detrimental effects on the operational properties of the perforated membrane (9), such as stability or throughput. Therefore, even though the drawings show a coaxial arrangement, deviations from such coaxial arrangement are not outside the scope of the invention.

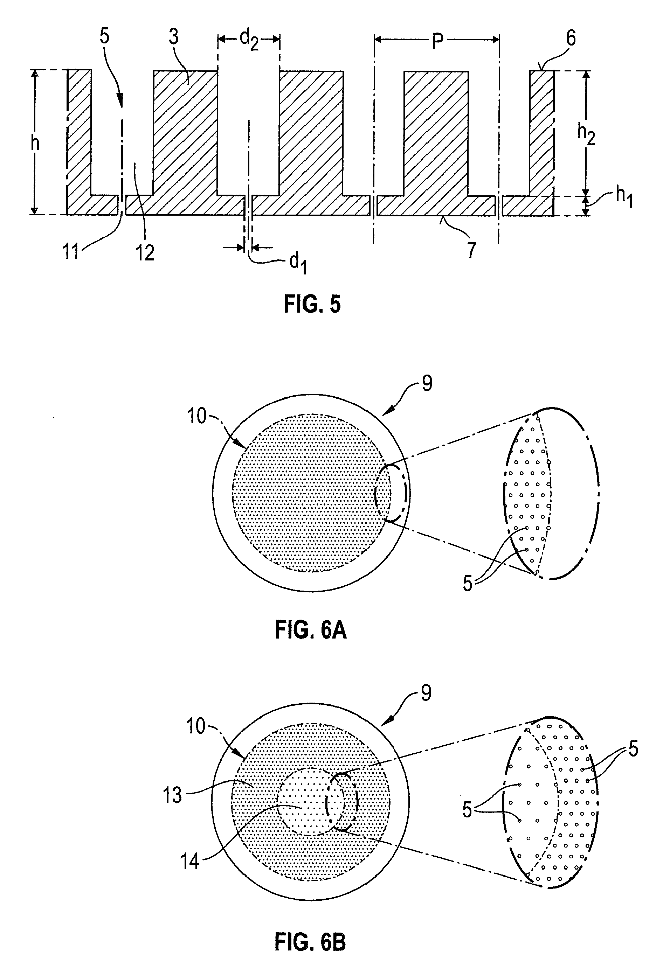

Figure 5 shows an enlarged cross-section of the region (10) exhibiting at least 50 holes/mm² of an exemplary embodiment of the perforated membrane (9) according to the invention. The holes (5) formed through the membrane which is primarily prepared from an electro-formable material (3), are spaced apart with a pitch (P), or inter-hole distance, and exhibit two different longitudinal sections (11, 12), which are coaxially arranged adjacent to one another (as indicated by the common dashed centre line). The first longitudinal section (11) has first diameter (d₁) and a first length, or depth, (h₁) and is located at the external face (7), or downstream side; i.e. the face or side, of the perforated membrane (9) which, during operation in an inhalation device such as a vibrating mesh nebuliser, faces away from the liquid to be nebulised and from which the liquid to be nebulised is emitted as a nebulised aerosol. The second longitudinal section (12) has second diameter (d₂) and a second length, or depth, (h₂) and is located at the internal face (6), or upstream side; i.e, the face or side, of the perforated membrane (9) which, during operation in an inhalation device such as a vibrating mesh nebuliser, faces the liquid to be nebulised and/or is in direct contact with said liquid. The second diameter (d₂) is larger than the first diameter (d₁), and the second length (h₂) is larger than the first length (h₁). In other words, the holes (5) comprise a first, relatively narrow and short segment (the first longitudinal section (11)) that abruptly broadens into a second wider, longer segment (the second longitudinal section (12)). This specific shape of the holes (5) may, for example, be achieved by the process according to the invention which will be described in further detail below.

The dimensions of the second longitudinal segment of the hole, which is larger than the first segment in diameter and length, are believed to be less critical for the aerosol quality and output rate. The second length (h₂), i.e. the length of the second longitudinal segment, may be selected as required to achieve the required overall thickness and stability of the perforated membrane (9). For reasons of process economics and/or vibrational properties, though, the length (h₂) of the second segment is typically smaller than 100 µm, depending however on the material from which the membrane is made. Optionally, the length (h₂) of the second segment is from about 30 µm to about 100 µm, such as from about 40 µm to about 80 µm.

The diameter of the second segment, i.e. the second diameter (d₂), is optionally in the range from about 5 µm to about 50 µm, or from about 5 µm to about 40 µm, or from about 10 µm to 35 µm, respectively. Since the second diameter (d₂) is relatively large, liquid typically enters the wider second segment easily, and it is believed that this segment has only minor impact on the liquid flow, but substantial impact on the stability of the membrane.

The dimensions of the first longitudinal segment, which is smaller than the second longitudinal segment and positioned downstream thereof, is believed to be more critical than those of the second segment in terms of aerosol particle size and output rate. Optionally, the diameter (d₁) of the first segment is in the range from about 1.5 µm about to 4.5 µm, such as about 1.5 µm, about 2 µm, about 2.5 µm, about 3 µm, about 3.5 µm, about 4 µm, or about 4.5 µm, respectively. The length (h₁) of the first longitudinal segment is optionally from about 4 µm to about 15 µm, or from about 5 µm to about 10 µm, respectively.

In a specific embodiment, the first diameter (d₁) is in the range from about 1.5 µm to about 4 µm, the first length (h₁) is in the range from about 5 µm to about 10 µm, and the second diameter (d₂) is in the range from about 20 µm to about 30 µm. In one particular embodiment, the perforated membrane (9) has a first longitudinal section (11) with a first diameter (d₁) in the range from about 2 µm to about 3 µm and a first length (h₁) in the range from about 5 µm to about 10 µm, and a second longitudinal section (12) with a second diameter (d₂) in the range from about 23 µm to about 27 µm. In a further embodiment, the perforated membrane (9) has a first longitudinal section (11) with a first diameter (d₁) of about 3 µm and a first length (h₁) of about 8 µm, and a second longitudinal section (12) with a second diameter (d₂) of about 25 µm.

The thickness of the perforated membrane (9), which may be approximately the sum of the first length (h₁) and of the second length (h₂), may optionally be in the range from about 35 µm to about 120 µm, such as from about 40 µm to about 100 µm.

The perforated membrane (9) according to the invention may be made from any biocompatible material resilient enough to withstand the conditions during use, e.g. the vibrations in a vibrating mesh nebuliser. It may e.g. be made from stainless steel, ceramics, plastics, or electro-formable materials such as metals. This includes e.g. nickel, palladium, platinum, gold, manganese, rhodium, silver, copper, cobalt, tin, zinc, lead and their alloys. In particular, the perforated membrane (9) may be made from a metal selected from the group of nickel, palladium, cobalt or an alloy of at least two members thereof. As will be described in further detail below, the perforated membrane (9) according to the invention may be prepared in a two-step electroforming process; i.e. in this case the membrane comprises a first and a second electroformed layer. In one embodiment, both the first and the second layer may be made from the same material; e.g. both layers may be prepared from nickel. Alternatively, the first and the second layer may be made from different materials; e.g. the first layer may be made from a nickel-palladium alloy whereas the second layer may be made from nickel.

In one embodiment, the perforated membrane (9) has a region (10) with a hole density in the range from 100 to 400 holes/mm². Optionally, the hole density is at least 150 holes/mm². Optionally, the hole density is at least 200 holes/mm². Such high hole densities can only be achieved with a very small pitch (P), which is hardly feasible for perforated membranes obtained from the prior art 'thin resin' processes as described above.

In one embodiment, the region (10) comprising holes may be approximately circular and positioned centrally around the centre of the perforated membrane (9), as depicted in figures 6A and 6B. In one particular embodiment, the hole density may be uniform throughout the complete region (10), as shown in figure 6A. This may contribute to a homogeneous distribution of vibrations and thus improve the membrane's stability.

Alternatively, the hole density may vary within different areas of the region (10). For example, it may comprise a circular centre part (14) and an annular outer part (13), as show in figure 6B, wherein the centre part (14) exhibits a lower hole density than the outer part (13). This has been believed to provide increased membrane stability. However, the difference between the two or more hole densities has to be chosen carefully in order not to create weak spots or breaking points of the membrane, e.g. at the borderline between circular centre part (14) and annular outer part (13). In one specific embodiment, the perforated membranes (9) according to the invention may comprise a circular centre part (14) which exhibits no holes (5) at all. In this case, the circular centre part (14) preferably) has a diameter of about 0.5 mm or less in order to not reduce the throughput of the perforated membrane (9).

Other shapes and/or partitions of the region (10) or its areas are feasible. For example the region (10) may be elliptical, hexagonal, or polygonal, or there may be more than two areas of different hole densities within the region (10), and/or grid-shaped areas of lower hole density.

The pitch (P) is preferably selected to be not more than about 120 µm. Optionally, the pitch (P) is less than about 110 µm, or less than about 100 µm, or less than about 90 µm, or less than about 80 µm, or less than about 70 µm; or in the range from about 30 µm to about 120 µm, or from about 40 µm to about 100 µm, or from about 40 µm to about 80 µm, respectively. Optionally, the pitch (P) is equal to the thickness (h) of the membrane or less.

In one embodiment, the perforated membrane according to the invention has ratio of the pitch (P) to the first length (h₁) in the range of 2.5 to 25.

In a further embodiment, the perforated membrane (9) according to the invention comprises a region (10) which comprises only holes (5) of substantially the same first diameter (d₁), within the typical variabilities of the manufacturing processes for such membranes. This is useful if the aim is to produce aerosols with a narrow droplet size distribution, such as aerosols characterized by a geometric standard deviation (GSD) of less than about 1.3 or even less than about 1.2.

However, due to regulatory requirements it may in some instances be desirable to obtain slightly broader size distributions, for example to achieve aerosols that are comparable to those produced by certain conventional nebulisers. Therefore, the region (10) may alternatively comprise at least two different sets of holes (5) having different first diameters (d₁). For instance, the region (10) could comprise a first set of holes (5) having a first diameter (d₁) of about 2 µm and second set of holes (5) having a first diameter (d₁) of about 3.5 µm. The sets of holes (5) can be distributed uniformly throughout the whole region (10). Alternatively, they can be arranged in separate areas of the region (10). In a particular embodiment, the values of both the hole density and the first diameter (d₁) are varied in order to obtain a specific, desired droplet size distribution.

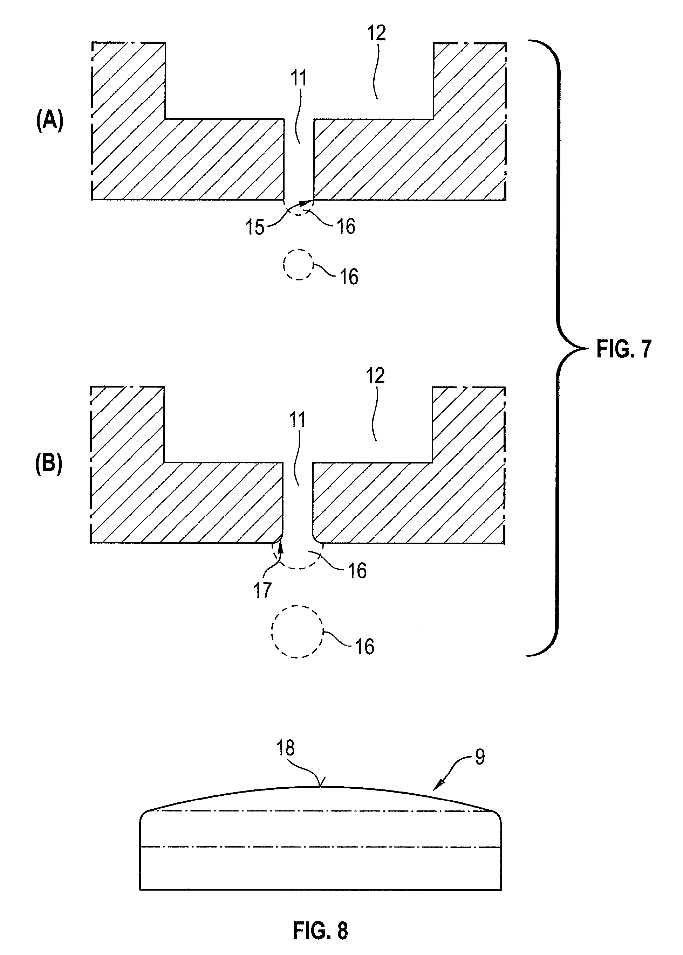

In a further embodiment, the holes (5) exhibit a sharp edge at least at their downstream end, i.e. on the external face (7). As will be described below, the specific preparation process of the perforated membrane (9) according to the invention exhibits cylindrical holes (5) at the external face (7) such that the first longitudinal section (11) of the hole (5) is shaped as a right circular cylinder which forms a 90° angle with the tangential plane of the external face (7). The edge of the first longitudinal section (11) at the tangential plane of the external face (7) is substantially free of roundings, splays, chamfers or imperfections like protruding ridges and/or indentations as illustrated by the sharp edge (15) in figure 7A. Like this, droplets of very precise volume and diameter are formed, because the droplets hardly spread at the edge. Moreover, a sharp edge at the downstream end has been found to reduce the undesirable dripping of liquid through the non-vibrating membrane.

The inventors found that surprisingly small droplet sizes (in VMD; volume median diameter, as measured by laser diffraction) could be achieved using a perforated membrane (9) according to the invention in combination with an aerosol generator such as the one disclosed in WO 2008/058941 A1 which is incorporated herein by reference. In prior art devices, the diameter of the droplets is typically about two times the diameter of the hole, an effect likely caused by the Plateau-Rayleigh instability which breaks up fluid jets into droplets upon exiting nozzles. For example, it was found by the inventors that the inhalation device, Aeroneb Go® by Aerogen, yields 2 to 10 µm droplets from tapered holes of 1 to 10 µm diameter at the narrowest point of taper. With Pari's eFlow^{®} device, droplet sizes of around 5.5 µm were obtained with 3 µm holes (ratio of droplet size to hole size 1.83), which could be reduced to droplet sizes of 4.5 µm with the same 3 µm holes when applying underpressure to the reservoir of liquid (ratio of droplet size to hole size 1.5).

Surprisingly, with the perforated membranes (9) according to the invention, even lower ratios of droplet size to hole size can be achieved, even at high throughput rates larger than 0.5 ml/min, such as about 1 ml/min, as shown in table 2 below.

**Table 2**

| Hole diameter [d₁ in µm] | VMD [µm] | | Droplet-to-hole ratio |
|---|---|---|---|
| | Mean | SD | |
| 2.0 | 2.29 | 0.07 | 1.15 |
| 2.5 | 2.92 | 0.09 | 1.17 |
| 2.5 | 3.46 | 0.17 | 1.38 |
| 3.0 | 4.35 | 0.19 | 1.45 |

It should be noted that, while in many therapeutic applications high throughput rates and in consequence rather dense aerosols are desirable in order to shorten the duration of inhalative treatments, there may be instances where aerosols with a lower density may be required for example when treating children. This may be achieved without replacing the perforated membrane (9) simply by adjusting e.g. the input power to the piezo element.

As mentioned, in one of the preferred embodiments, the holes (5) exhibit a substantially sharp edge at least at their downstream end, or at the external face (7) of the membrane. It is noted that the respective edges in most prior art membranes are not sharp. When the edge of the holes (5), in particular the edge of the first longitudinal section (11) at the external face (7), is less sharply defined, the droplet are subjected to spreading as illustrated at the position of the edge with roundings, splays, chamfers or imperfections (17) in figure 7B. This leads to larger droplets of a less precisely defined volume and may even cause the wetting of the external face (7) with the liquid formulation to be nebulised. In cases of low pitch values, larger droplets may lead to an increased risk of droplet coalescence at the external face (7), unpredictably broadening the droplet size distribution. Therefore, a sharp edge definition may be useful to reduce the risk of droplet coalescence at the external face (7), reduce dripping, and ensure a narrow size distribution with low geometric standard deviations (GSD). In particular, if the holes (5) have a first diameter (d₁) of about 4 µm or less and the pitch is below about 100 µm, it is preferred that the holes exhibit a sharp edge at their downstream ends.

In a further embodiment, the perforated membrane (9) at least partially forms a dome (18) in a central position, such that the dome (18) comprises the region (10). The dome (18) can be formed by any means known to a person skilled in the art, such as cold-forming after the preparation of the membrane itself, using for example the process according to the invention as will be detailed further below. The dome (18) may be used to improve the divergence of droplets ejected from the holes (5) of the perforated membrane (9) at the external face (7), and may further improve the stability of the perforated membrane (9). In a particular embodiment, a perforated membrane (9) with a diameter of about 7.6 mm is cold-formed into a so-called crown cap shape, thereby leaving a diameter of about 6 mm across which a dome (18) of about 0.3 to 0.4 mm height (measured at its centre) is formed, as illustrated in figure 8. The substantially straight sides of this 'crown cap' may e.g. be used to insert the perforated membrane (9) into a piezoelectric transducer body and affix it there by e.g. gluing or welding.

In another embodiment, the perforated membrane (9) further comprises a water-repellent, biocompatible and/or corrosion resistant coating (not depicted). Such coatings may either be applied to only one side, or face, of the perforated membrane (9) or to both sides, or faces; and may be made from any bio-compatible material providing water-repellent and/or corrosion resistant properties to the perforated membrane (9) or improving its bio-compatibility; e.g. ceramics, metals and/or polymers (e.g. PTFE; polytetrafluoroethylene or parylene). In a specific embodiment, the perforated membrane (9) may comprise an electroformed gold coating to improve bio-compatibility. Application of such coatings may be performed by any means known from prior art. For instance, US5152456A describes a water-repellent coating of PTFE particles applied and heat-treated in situ to form a closed coating; alternatively the PTFE particles may be applied autocatalytically in a nickel-phosphorous matrix to provide water-repellence and corrosion resistance. Parylene is typically applied by chemical vapour deposition. WO03/028895A2 and US6427682B1 describe perforated membranes (9) with two electroformed layers; one may e.g. be made of inexpensive nickel, nickel alloys (e.g. nickel manganese), copper or tin; while the other may e.g. be made of a more corrosion resistive material such as a palladium, palladium-nickel-alloys, palladium-cobalt-alloys, gold, nickel-gold alloys, platinum, rhodium or silver.

Applying the water-repellent, bio-compatible and/or corrosion resistant coating to the perforated membrane (9) onto its external face (7) in a subsequent electro-forming step (i.e. following the preparation steps described in claim 14) offers the advantage that one can further reduce the first diameter (d₁) of the holes (5) and hence the droplet size of the aerosol. However, it should be considered that such a subsequent coating step may decrease the precision of the hole diameter (d₁) and cause the edges to gradually turn from the desirable sharp edges (15), as illustrated in figure 7A, to rounded edges or edges with imperfections like protruding ridges that resemble the edges (17) depicted in figure 7B.

In one specific embodiment of the invention, the perforated membrane (9), about 7.6 mm in diameter, comprises a region (10), 4.7 mm in diameter, with a hole density larger than 230 holes/mm², each hole (5) having a first longitudinal section (11) being shaped as a right circular cylinder with a first length (h₁) of 8 µm and a first diameter (d₁) of 3 µm ± 0.5 µm, and an adjacent, substantially coaxially arranged second longitudinal section (12) being shaped as a right circular cylinder with a second length (h₂) of 50 µm and a second diameter (d₂) of 25 µm ± 3 µm. The holes are spaced at a pitch of 70 µm. The thickness (h) of the perforated membrane (9) is 58 µm +2/-4 µm. The membrane (9) may exhibit a dome with a height of 0.3 to 0.4 mm spanning across a diameter of about 6 mm, such as illustrated in figure 8. The aspect ratio of the first length (h₁) divided by the first diameter is at least 2 (d₁) is approximately 2.67. Optionally, the perforated membrane (9) may be made from nickel.

In another specific embodiment of the invention, the perforated membrane (9), 7.6 mm in diameter, comprises a region (10), about 4.7 mm in diameter, with a hole density larger than 115 holes/mm², each hole (5) having a first longitudinal section (11) being shaped as a right circular cylinder with a first length (h₁) of 8 µm and a first diameter (d₁) of 3 µm +0.0/-0.5 µm, and an adjacent, substantially coaxially arranged second longitudinal section (12) being shaped as a right circular cylinder with a second length (h₂) of 50 µm and a second diameter (d₂) of 25 µm ± 3 µm. The holes are spaced at a pitch of 100 µm. The thickness (h) of the perforated membrane (9) is 58 µm + 2/-4 µm. The membrane (9) may exhibit a dome with a height of 0.3 to 0.4 mm spanning across a diameter of about 6 mm, such as illustrated in figure 8. The aspect ratio of the first length (h₁) divided by the first diameter is at least 2 (d₁) is approximately 2.67. Optionally, the perforated membrane (9) may be made from nickel.

In another specific embodiment of the invention, the perforated membrane (9), 7.6 mm in diameter, comprises a region (10), about 5.5 mm in diameter, having a circular centre part (14), about 1.0 mm in diameter, with a hole density larger than 100 holes/mm²; and an annular outer part (13) with a hole density larger than 375 holes/mm², each hole (5) having a first longitudinal section (11) being shaped as a right circular cylinder with a first length (h₁) of 8 µm and a first diameter (d₁) of 3 µm ± 0.5 µm, and an adjacent, substantially coaxially arranged second longitudinal section (12) being shaped as a right circular cylinder with a second length (h₂) of 50 µm and a second diameter (d₂) of 25 µm ± 3 µm; wherein the holes are spaced at a pitch of 100 µm in the circular centre part (14) and at a pitch of 50 µm in the annular outer part (13). The thickness (h) of the perforated membrane (9) is 58 µm + 2/-4 µm. The membrane (9) may exhibit a dome with a height of 0.3 to 0.4 mm spanning across a diameter of about 6 mm, such as illustrated in figure 8. The aspect ratio of the first length (h₁) divided by the first diameter is at least 2 (d₁) is approximately 2.67. Optionally, the perforated membrane (9) may be made from nickel.

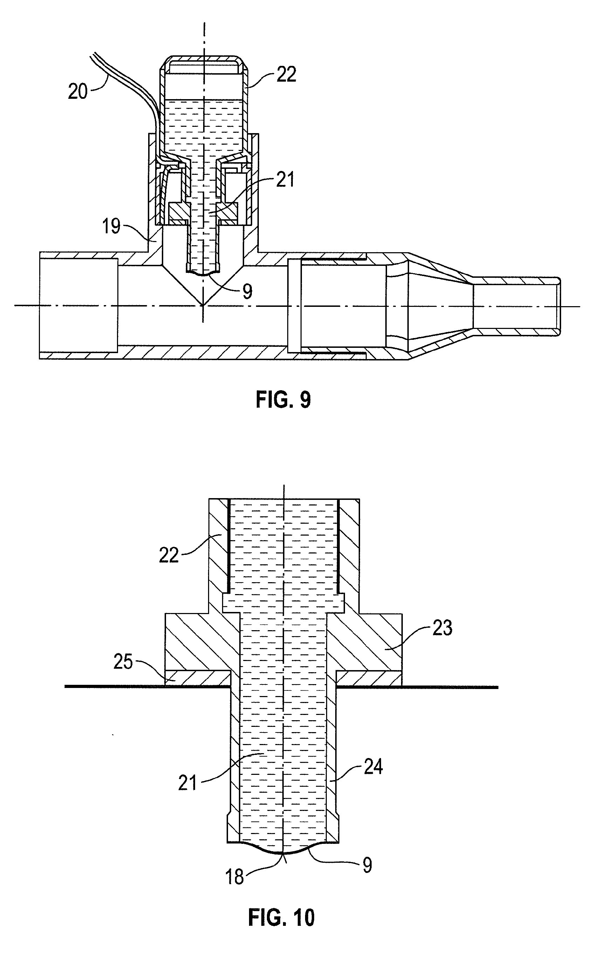

The invention further provides an inhalation device (19) comprising the above mentioned perforated membrane (9) as depicted in figure 9. The cross-section shows an inhalation device (19) comprising a power cable (20), a liquid reservoir (21) holding the liquid formulation to be nebulised and a nebulising means (22) which is equipped with a perforated membrane (9) according to the invention. In a particular embodiment, an inhalation device (19) is provided, wherein the perforated membrane (9) is coupled mechanically and acoustically to a piezoelectric transducer. Such an embodiment is illustrated in figure 10, where the nebulising means (22) comprises a perforated membrane (9) with a dome (18), a piezoelectric transducer body with a thicker-walled stress concentration zone (23) and a thinner-walled vibration displacement amplification zone (24) and an annular piezo-element (25). An exemplary embodiment of the nebulising means (22) is disclosed in US2010044460A1, the content of which is incorporated herein in its entirety. The perforated membrane (9) is attached to the vibration displacement amplification zone (24) of the piezoelectric transducer body in such a way that it is mechanically and acoustically coupled, thereby yielding an 'active' perforated membrane (9) which actually vibrates in response to the vibrations transferred from the piezoelectric transducer body. Of the various options to mechanically couple the perforated membrane (9) to the piezoelectric transducer body, gluing, brazing and welding (e.g. laser welding) are preferred in order to ensure proper acoustic coupling. Other options for mechanical coupling, such as crimping or link members (e.g. threaded means for wedging the membrane on the transducer; alone or in combination with washers and/or spring coils) are feasible as well but should be evaluated more carefully with regard to their acoustic coupling performance; e.g. whether the vibrations of the piezo-element (25) are sufficiently transferred to the perforated membrane (9) to induce active vibration.

The invention further provides the use of the above mentioned perforated membrane (9) or of the above mentioned inhalation device (19) for inhalation therapy.

The invention further provides a process for preparing a perforated membrane (9) as described above by electro-forming. The process comprises the steps of
(A) coating a silicon wafer (26) with a conductive seed layer (27),
(B) preparing a first patterned photoresist layer (29) by
   i) spin coating the product of step (A) with a thin layer of a first photosensitive material (28) having a first thickness,
   ii) exposing said first photosensitive material (28) to UV-light through a laser-cut mask (8) to solidify the first photosensitive material (28) in a first predetermined pattern
   iii) chemically developing the first photosensitive material (28) to obtain a predetermined pattern of first right circular cylinders having a first diameter and a first length
(C) preparing a first layer (30) of the perforated membrane (9) by electro-depositing an electro-formable material (3) using the first patterned photoresist layer (29) of step (B) until the thickness of said first layer (30) approximately reaches the first length of the photoresist cylinders,
(D) preparing a second patterned photoresist layer (32) by
   iv) laminating the product of step (C) with a layer of a second photosensitive material (31) having a second thickness larger than the thickness of the layer of the first photosensitive material (28)
   v) exposing said second photosensitive material (31) to UV-light through a laser-cut mask (8) to solidify the second photosensitive material (31) in a second predetermined pattern
   vi) chemically developing the second photosensitive material (31) to obtain a predetermined pattern of second right circular cylinders adjacent to the first right circular cylinders and having a second length and a second diameter,
      wherein said second length is larger than the first length and said second diameter is larger than the first diameter, and wherein the first and second right circular cylinders are arranged in such a way that the base of the first right circular cylinder facing the second right circular cylinder is contained in the base of the second right circular cylinder facing the first right circular cylinder
(E) rinsing the product of step (D) with acid
(F) preparing a second layer (33) of the perforated membrane (9), adjacent to the first layer (30), by electro-depositing an electro-formable material using the second patterned photoresist layer (32) of step (D) until the thickness of said second layer (33) approximately reaches the second length of the photoresist cylinders,
(G) removing the silicon wafer (26) along with the conductive seed layer (27),
(H) removing the photoresist materials (28, 31) in a dry chemical etching process.

Following this preparation process, the obtained perforated membrane (9) is preferably subjected to an ultrasonic cleaning step in the presence of wetting agents.

The described process according to the invention differs from the one disclosed in US7972543B2 in that the holes with the smaller diameter are formed in the first step, i.e. with the first thinner layer of photosensitive material (28) applied directly onto the electrically conductive substrate (i.e. the silicon wafer (26) with the conductive seed layer (27)), whereas in US7972543B2 they are formed in the second step with the second thicker layer of photosensitive material applied onto the first layer of photosensitive material. This difference in the described process according to the invention has various advantageous technical effects.

First of all, higher precisions of the hole diameter (d₁) at the external face (7), and in consequence droplet size, will be achieved when forming the holes (5) with the smaller diameter (d₁) with the thinner layer of photosensitive material. As described earlier, e.g. by EP1199382A1, it was empirically found in 'front exposure' processes that the preparation of stable photoresist columns with reproducible size and shape gets increasingly difficult when the column's height-to-diameter ratio exceeds a factor of one. Using the process of the present invention, said factor was increased to at least 2, preferably at least 2.5 or more. Still, a thickness of the photosensitive material layer not larger than 20 µm (i.e. photoresist columns not higher than 20 µm) will be beneficial with regard to precision when aiming for hole diameters of only about 1 µm to about 5 µm. In contrast, in US7972543B2, the holes with the smaller diameter are formed with a 150 µm to 160 µm thick layer of photosensitive material, and thus would not allow for precise hole diameters in the range of 1 µm to 5 µm. This may be attributed to the fact that it was not the aim of US7972543B2 to provide droplet sizes suitable for inhalation but to provide a through-holed metal structure through which a continuous phase could be passed in order to emulsify it. Thus, far larger hole dimensions of e.g. 10 x 40 µm (square), 20 x 20 µm (square) or 10 x 15 µm (elliptical) are sufficient in US7972543B2 while being completely unsuitable for inhalation purposes.

The precision of the hole diameter is improved further by employing a liquid negative photosensitive material (28) to form the first patterned photoresist layer (29). While dry negative photosensitive materials, so called dry films (as used for the second patterned photoresist layer (32)) are typically easier to apply, they rarely allow for hole diameters smaller than 10 µm.

Secondly, the surface of e.g. a silicon wafer (26) is highly polished and far smoother - even after coating it with a thin conductive seed layer (27) and a thin chromium layer (28) - than the surfaces obtainable with electro-deposition. The edge definition at the external face (7) is thus improved when forming the holes with the smaller diameter first, using the layer of photosensitive material which is applied directly onto the smooth electrically conductive substrate, i.e. sharper edges are obtained. In US7972543B2, the edge definition of the holes with the smaller diameter will not be as sharp as with the process according to the present invention since it is highly dependent on the properties of the electrodeposited layer which will serve as the external face. This is also the reason, why in the present invention silicon wafers are preferred over e.g. glass or stainless steel substrates. While the latter are equally common in electrodeposition processes of the state of the art, they do not provide the same surface smoothness as silicon.

A further advantage is the improved precision of the inner shape of the holes using the process according to the present invention. In US7972543B2, the first layer of photosensitive material is used to form the holes with the larger diameter. This results in a larger reduction of the conductive area of the electrically conductive substrate because a larger fraction of said substrate surface will be 'occupied' by the non-conductive photoresist columns. When applying e.g. the second, round, smaller photoresist column onto the first, round, larger one, the remaining annular area at the transition from larger to smaller column diameter is still non-conductive. In consequence, said annular, non-conductive area will at first not receive any electro-formable material during the second electrodeposition step. Only after the process progresses, electro-formable material will gradually 'overflow' over the annular area as well to fill the gap at the transition from larger to smaller diameter of the photoresist column. This leads to less precisely defined inner shapes of the holes. In contrast, with the process according the invention (i.e. when forming the smaller diameter photoresist columns first and applying the larger diameter photoresist columns on top, instead of vice versa) all the area not 'occupied' by the larger diameter photoresist columns remains conductive and will immediately receive electro-formable material during the second electrodeposition step. This provides a sharp edge at the transition from smaller to larger diameter of the photoresist column, or from first longitudinal section (11) to second longitudinal section (12); and hence a more precisely defined and controlled inner shape of the holes (5) in the perforated membrane (9). This ensures more homogenous flow of liquid and droplet size distribution.

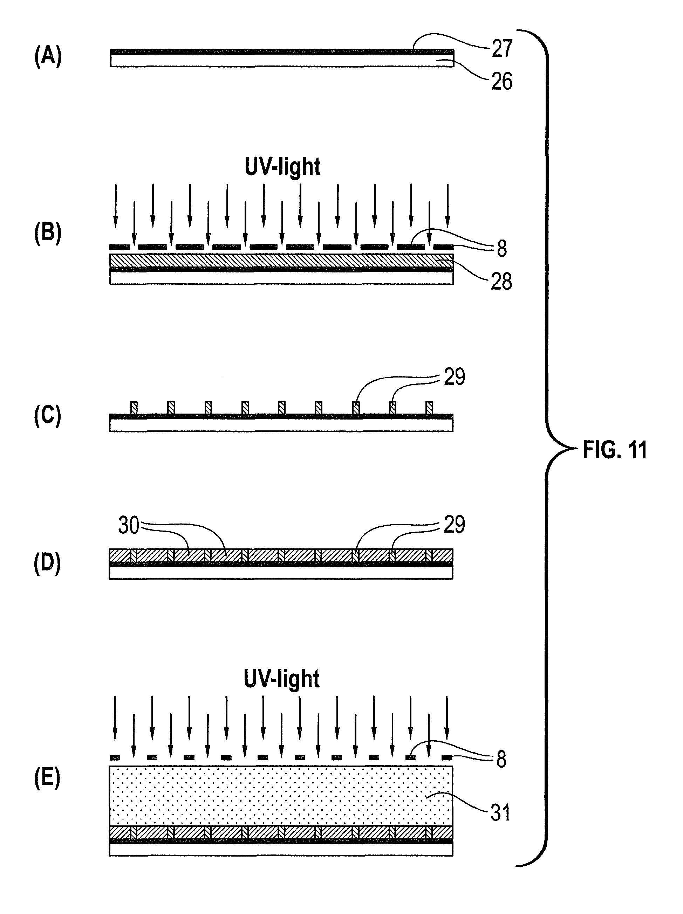

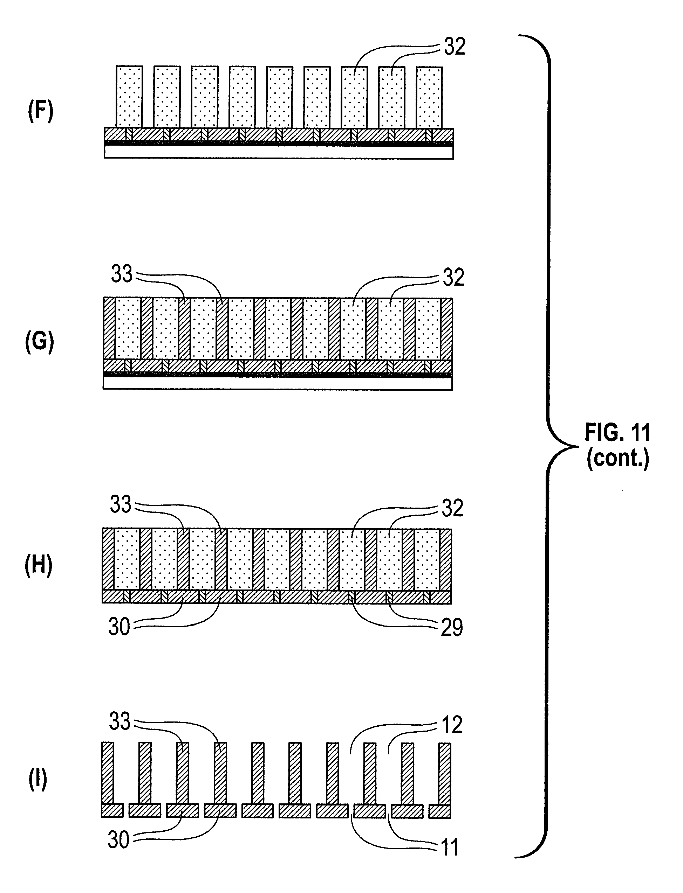

In one embodiment of the invention, a perforated membrane (9) is prepared by a process as illustrated in figures 11A to 11I. A silicon wafer (26) is coated with a conductive seed layer (27). The conductive seed layer (27) may be applied by any sputtering or chemical vapour deposition (CVD) processes known to a person skilled in the art. The conductive seed layer (27) may further be a composite of two or more separate layers made from materials which provide for adhesion (to the silicon wafer (26) and/or the electro-formable material (3)), conductivity and/or improved peeling (when the silicon wafer (26) and the conductive seed layer (27) get removed). Said materials may be selected from chromium, titanium, silver, copper, gold and/or aluminium. For example, the conductive seed layer (27) may be a composite of:
a chromium layer and a copper layer, or
a titanium layer and a copper layer, or
a chromium layer and a gold layer, or
a titanium layer and a gold layer, or
a chromium layer and a silver layer, or
a titanium layer and a silver layer, or
a chromium layer and an aluminium layer, or
a titanium layer and an aluminium layer, or
a chromium layer, a copper layer and another chromium layer, or
a titanium layer, a copper layer and another titanium layer, or
a chromium layer, a copper layer and a titanium layer, or
a titanium layer, a copper layer and a chromium layer, or
a chromium layer, a gold layer and another chromium layer, or
a titanium layer, a gold layer and another titanium layer, or
a chromium layer, a gold layer and a titanium layer, or
a titanium layer, a gold layer and a chromium layer, or any similarly arranged composites providing for adhesion, conductivity and/or improved peeling. In the above mentioned examples of the conductive seed layer (27), the order in which the materials are listed is to be interpreted as the order in which the materials are applied to the silicon wafer (26); i.e. the first listed material is applied directly onto the silicon wafer (26), the second listed material is applied onto the first listed, and so on.

After the preparation of the conductive seed layer (27), the first patterned photoresist layer (29) is prepared by spin coating this electrically conductive substrate with a thin layer of a first photosensitive material (28); for example a liquid negative photosensitive material chosen from SU-8, AR-N 4400 or AZ9260, until reaching a first thickness ranging from 5 µm to 20 µm. The liquid negative photosensitive material is then exposed to UV-light (365 nm or 405 nm) through a laser-cut mask (8) to solidify it into the first patterned photoresist layer (29) with a first predetermined pattern. The energy density is set from 250 to 500 mJ/cm², preferably from 300 to 400 mJ/cm². The unexposed fractions of the liquid negative photosensitive material are then removed in a chemical developing step, using the developer indicated in the data sheet of the respective liquid negative photosensitive material, in order to obtain the first predetermined pattern of first right circular cylinders having a first diameter in the range of 1 µm to 5 µm, preferably 1.5 µm to 4 µm; and a first length not larger than 20 µm, preferably 5 µm to 10 µm. In a first electrodeposition step, a first layer (30) of the perforated membrane (9) is formed from an electro-formable material (3), for example nickel or a nickel-palladium alloy, until the thickness (h₁) of said first layer (30) approximately reaches the first length of the photoresist cylinders.

Using markers in order to align first and second photoresist columns substantially coaxially to one another, the second patterned photoresist layer (32) is prepared by laminating a layer of a second photosensitive material (31); for example a dry negative photosensitive material chosen from Ordyl FP400 or Ordyl SY300. Said laminated layer has a second thickness larger than the thickness of the layer of the first photosensitive material layer (28); e.g. 50 µm, but typically not larger than 100 µm. The dry negative photosensitive material is then exposed to UV-light (365 nm or 405 nm) through a laser-cut mask (8) to solidify it into the second patterned photoresist layer (32) having a second predetermined pattern. The energy density is set from 250 to 500 mJ/mm², preferably from 300 to 400 mJ/cm². The unexposed fractions of the dry negative photosensitive material are then removed in a chemical developing step, using the developer indicated in the data sheet of the respective dry negative photosensitive material, in order to obtain the second predetermined pattern of second right circular cylinders having a second diameter in the range of 4 µm to 50 µm, preferably 20 µm to 30 µm, e.g. 25 µm; and a second length typically not larger than 100 µm.

Prior to the second electrodeposition step, the surface is rinsed with acid; for example an acid chosen from sulphuric acid or sulphamic acid, in order to 'activate' the surface by removing oxidised nickel and thus improving the adhesion of the second layer (33) to the first layer (30). Then, a second layer (33) of the perforated membrane (9) is formed adjacent to the first layer (30) by electro-depositing electro-formable material (3); for example the same nickel or the same nickel-palladium alloy as used for the formation of the first layer (30), until the thickness (h₂) of said second layer (33) approximately reaches the second length of the photoresist cylinders. In an alternative embodiment, the first and second layer (30, 33) may be made from different electro-formable materials (3); e.g. the first layer (30) from a nickel-palladium alloy and the second layer (33) from nickel.

Finally, the silicon wafer (26) is removed along with the conductive seed layer (27) in a mechanical peeling step. Preferably, the conductive seed layer (27) is prepared such that its adhesion to the silicon wafer (26) is stronger than its adhesion to the first layer (30) of electro-formable materials (3), in order to allow the perforated membrane (9) to be lifted off the substrate without damaging the sharp edges (15) of the holes (5) at the external face (7). Then both solidified negative photoresist materials (29, 32) are removed in a dry chemical etching process; for example an oxygen based plasma stripping process as known from the state of the art. Optionally, wet chemical etching processes could also be employed for the removal of the photoresist material. However, due the higher removal precision of gaseous removers (such as oxygen plasma) in comparison with liquid removers, the oxygen based plasma stripping process is usually preferred.

It will be apparent to those skilled in the art that the present description is a disclosure of exemplary embodiments only, and that various modifications and variations can be made in the present invention without departing from the scope or spirit of the invention. For instance, features illustrated or described as part of one embodiment, can be used on another embodiment to yield a still further embodiment. Thus, it is intended that the present invention covers such modifications and variations within the scope of the appended claims and their equivalents.

## Claims

1. A perforated membrane comprising a region with at least 50 holes/mm², each hole having a first longitudinal section being shaped as a right circular cylinder with a first length and first diameter, and an adjacent second longitudinal section being shaped as a right circular cylinder with a second length and second diameter,
wherein the first and second longitudinal sections are arranged in such a way that the base of the first longitudinal section facing the second longitudinal section is contained in the base of the second longitudinal section facing the first longitudinal section;
wherein the second length is larger than the first length and the second diameter is larger than the first diameter;
wherein the first diameter is from 1 µm to 5 µm and the first length is not more than 20 µm; wherein the second diameter is from 4 µm to 50 µm; and
wherein the aspect ratio of the first length divided by the first diameter is at least 2.

2. The perforated membrane according to claim 1, wherein the aspect ratio of the first length divided by the first diameter is at least 2.5.

3. The perforated membrane according to claim 1 or 2 being made from a metal selected from the group of nickel, palladium, cobalt or an alloy of at least two members thereof.

4. The perforated membrane according to any of the preceding claims, wherein the first diameter is in the range from 1.5 µm to 4 µm and the first length is in the range from 5 µm to 10 µm, and
wherein the second diameter is optionally in the range from 20 µm to 30 µm.

5. The perforated membrane according to any of the preceding claims, wherein the hole density in said region is in the range from 100 to 400 holes/mm².

6. The perforated membrane according to any of the preceding claims, wherein the ratio of the pitch to the first length in the range from 2.5 to 25; or
wherein the pitch is equal to the thickness of the membrane or less.

7. The perforated membrane according to any of the preceding claims, wherein the region comprises only holes of the same first diameter, or
wherein the region comprises at least two different sets of holes having different first diameters.

8. The perforated membrane according to any of the preceding claims, wherein the holes exhibit a sharp edge at their downstream end.

9. The perforated membrane according to any of the preceding claims, wherein said membrane at least partially forms a dome in a central position, and wherein the dome comprises said region.

10. The perforated membrane according to any of the preceding claims, wherein said membrane further comprises a water-repellent, bio-compatible and/or corrosion resistant coating.

11. An inhalation device comprising the perforated membrane according to claims 1 to 10.

12. The inhalation device according to claim 11, wherein the perforated membrane is coupled mechanically and acoustically to a piezoelectric transducer.

13. The use of the perforated membrane according to claims 1 to 10 or of the inhalation device of claim 11 or 12 for inhalation therapy.

14. A process for preparing the perforated membrane according to claims 1 to 10 by electro-forming, comprising the steps of
(A) coating a silicon wafer with a conductive seed layer,
(B) preparing a first patterned photoresist layer by
i) spin coating the product of step (A) with a thin layer of a first photosensitive material having a first thickness,
ii) exposing said first photosensitive material to UV-light through a laser-cut mask to solidify the first photosensitive material in a first predetermined pattern, and
iii) chemically developing the first photosensitive material to obtain a predetermined pattern of first right circular cylinders having a first diameter and a first length
(C) preparing a first layer of the perforated membrane by electro-depositing an electro-formable material using the first patterned photoresist layer of step (B) until the thickness of said first layer approximately reaches the first length of the photoresist cylinders,
(D) preparing a second patterned photoresist layer by
iv) laminating the product of step (C) with a layer of a second photosensitive material having a second thickness larger than the thickness of the layer of the first photosensitive material
v) exposing said second photosensitive material to UV-light through a laser-cut mask to solidify the second photosensitive material in a second predetermined pattern
vi) chemically developing the second photosensitive material to obtain a predetermined pattern of second right circular cylinders adjacent to the first right circular cylinders and having a second length and a second diameter, wherein said second length is larger than the first length and said second diameter is larger than the first diameter, and wherein the first and second right circular cylinders are arranged in such a way that the base of the first right circular cylinder facing the second right circular cylinder is contained in the base of the second right circular cylinder facing the first right circular cylinder
(E) rinsing the product of step (D) with acid
(F) preparing a second layer of the perforated membrane, adjacent to the first layer, by electro-depositing an electro-formable material using the second patterned photoresist layer of step (D) until the thickness of said second layer approximately reaches the second length of the photoresist cylinders,
(G) removing the silicon wafer along with the conductive seed layer,
(H) removing the photoresist materials in a dry chemical etching process.

15. The process according to claim 14,
wherein the conductive seed layer is a composite of two or more separate layers made of materials selected from chromium, titanium, silver, copper, gold and/or aluminium; and/or wherein the first photosensitive material is a liquid negative photosensitive material chosen from SU-8, AR-N 4400 or AZ9260; and/or
wherein the second photosensitive material is a dry negative photosensitive material chosen from Ordyl FP400 or Ordyl SY300; and/or
wherein the acid is chosen from sulphuric acid or sulphamic acid;
and/or wherein the dry chemical etching process is an oxygen based plasma stripping process.
